# EUROPÄISCHE PATENTSCHRIFT

(11) **EP 0 704 423 B1**
(45) Veröffentlichungstag und Bekanntmachung des Hinweises auf die Patenterteilung: **24.03.1999**
(21) Anmeldenummer: 95113599.5
(22) Anmeldetag: 30.08.1995
(51) Int. Cl.: C07C 67/04, C07C 69/63

(54) **Verfahren zur Herstellung von Chloressigsäure-tert.-butylester**
Process for the preparation of the tert-butyle ester of chloroacetic acid
Procédé de préparation de l'ester tert-butylique de l'acide chloroacétique

(30) Priorität: 27.09.1994 DE 4434444
(43) Veröffentlichungstag der Anmeldung: 03.04.1996
(73) Patentinhaber: Clariant GmbH, 65929 Frankfurt am Main (DE)
(72) Erfinder: Seidel, Andreas, Dr., D-50939 Köln (DE); Peters, Dieter, Dr., D-50354 Hürth-Efferen (DE); Weferling, Norbert, Dr., D-50354 Hürth-Efferen (DE); Mouratidis, Dimitrios, D-50226 Frechen (DE)

(56) Entgegenhaltungen:
- WO-A-91/16362

## Beschreibung

Chloressigsäure-tert.-butylester ist ein organisches Zwischenprodukt, das insbesondere zur Herstellung von Glycidestern aus Ketonen in der Darzens-Kondensation benutzt wird. Die entsprechenden Glycidester lassen sich ihrerseits durch Decarboxylierung in Aldehyde überführen, die ein Kohlenstoffatom reicher sind als das eingesetzte Keton (vgl. z.B. L. u. M. Fieser, Organische Chemie (1965), S. 575). Derartige Vergrößerungen eines Molekülgerüsts sind häufig Bestandteil organischer Synthesen, z.B. von Pharmazeutika.

Eine breite Verwendung des Chloressigsäure-tert.-butylesters ist jedoch mangels eines technisch attraktiven Herstellverfahrens bisher nicht möglich. In der einschlägigen Literatur sind lediglich Laborverfahren beschrieben, so z.B. die Veresterung von Chloressigsäure mit tert.-Butanol und Dicyclohexylcarbodiimid, katalysiert durch 4-Dimethylamino-pyridin, oder die Umsetzung von Chloracetylchlorid mit tert.-Butanol in Benzol in Gegenwart von aktiviertem Aluminiumoxid oder mit tert.-Butanol und Dimethylanilin. Weiterhin beschreiben z.B. W.S. Johnson et al., J. Amer. Chem. Soc. 75 (1953), S. 4998 die Addition von Chloressigsäure an Isobuten, katalysiert durch Schwefelsäure in Dioxan bei Raumtemperatur.

Alle genannten Verfahren stellen Anforderungen, die eine Übertragung in den technischen Maßstab erschweren. So werden kostspielige Hilfsstoffe stöchiometrisch eingesetzt, die in der Reaktion verbraucht und nicht zurückgeführt werden können, oder Katalysatoren benutzt, die die Verfahrensführung komplizieren.

Die günstigste Stoffbilanz der drei Verfahrensvarianten weist die Addition von Chloressigsäure an Isobuten auf, bei der weder Wasser noch Chlorwasserstoff entstehen. Nachteilig an diesem Verfahren ist, daß Schwefelsäure als Katalysator eingesetzt wird, welche die Di- und Trimerisierung von Isobuten katalysiert. Diese Nebenreaktionen führen beim genannten Verfahren zu nennenswerten Selektivitätseinbußen. Zur Entfernung der Schwefelsäure wird eine Neutralisation mit wäßriger Kalilauge durchgeführt.

Dieser Verfahrensschritt führt jedoch zum Verlust erheblicher Mengen nicht umgesetzter Chloressigsäure, die ebenfalls neutralisiert und in die wäßrige Phase überführt wird.

Es war daher die Aufgabe gestellt, ein technisch gangbares Verfahren für die Herstellung von Chloressigsäure-tert.-butylester zu finden, das die genannten Nachteile vermeidet.

Bei der Untersuchung der Addition von Chloressigsäure an Isobuten stellten wir überraschend fest, daß die Reaktion ohne Katalysator (wie z.B. Schwefelsäure), Hilfsstoffe und Lösemittel mit hohen Raumzeitausbeuten durchgeführt werden kann, wenn die Reaktionstemperatur angehoben wird. Das so modifizierte Additionsverfahren verläuft mit einer erstaunlich hohen Ausbeute, ber. auf umgesetztes Isobuten (sog. Isobuten-Selektivität) und bietet damit erhebliche Vorteile, die eine Übertragung in den technischen Maßstab ermöglichen. So vereinfacht sich die Auftrennung des Produktgemischs, da lediglich nicht umgesetzte Chloressigsäure aus der Reaktionslösung abgetrennt werden muß und eine weitere destillative Rektifikation des Produkts nicht erforderlich ist. Es hat sich hierbei erwiesen, daß die destillative Abtrennung nicht umgesetzter Chloressigsäure als Sumpfprodukt zu keinen nennenswerten Ausbeuteverlusten während der Destillation führt und ein lagerstabiles Produkt resultiert.

Im einzelnen betrifft die Erfindung nunmehr ein Verfahren zur Herstellung von Chloressigsäure-tert.-butylester durch Anlagerung von Chloressigsäure an Isobuten in einem geschlossenen Gefäß unter dem sich einstellenden Druck, welches dadurch gekennzeichnet ist, daß man die Reaktion in Abwesenheit von Katalysatoren und Lösemitteln in einem Druckbehälter bei einer Temperatur von 80° bis 110 °C unter dem sich einstellenden Druck von 3 bis 12 bar über einen Zeitraum von 1 bis 12, vorzugsweise 4 bis 8 Stunden durchführt, den Druckbehälter abkühlt, das gekühlte Produktgemisch entnimmt und es ohne vorherige Neutralisation unter vermindertem Druck destillativ aufarbeitet.

### Beispiel

189 g (2 mol) geschmolzene Chloressigsäure [94,5] wurden in einem mit einer PTFE-Hülse ausgekleideten Laborautoklaven, der mit einem PTFE-ummantelten, magnetgetriebenen Mehrflügelrührer ausgerüstet war, eingewogen. Durch Einbringen des Autoklaven in ein Eis/Kochsalz-Bad wurde der Autoklav abgekühlt und die Chloressigsäure eingefroren. Zeitgleich wurden in eine durch ein Trockeneis/Aceton-Kältebad gekühlte Vorlage etwa 112 g (1,98 mol) Isobuten [57,1] einkondensiert und in den vorgekühlten Autoklaven umgefüllt, wobei ein Teil des Isobutens verdampfte. Der Autoklav wurde verschlossen und auf 90 °C erwärmt, wobei sich ein Innendruck von anfangs 10 bar einstellte. Unter intensivem Rühren wurde die Reaktion bei 90 °C 6 Stunden fortgesetzt. Nach Abkühlung auf Raumtemperatur entnahm man dem Autoklaven 277,4 g Produktlösung und überführte sie in einen Rotationsverdampfer. Man destillierte unter einem Druck von 50 mbar und steigerte die Badtemperatur allmählich bis auf 90 °C.

Als Destillat gewann man 152,2 g Produkt mit 97,7 Gew% (= 989 mmol) Chloressigsäure-tert.-butylester [151,6] sowie Di- und Tri-isobuten (entsprechend 24 mmol Isobuten) und 26 mmol tert.-Butanol. Außerdem wurden in einer Trockeneis/Aceton-Kühlfalle 29,7 g nicht umgesetztes Isobuten aufgefangen, das 0,51 g Di- und Tri-isobuten (entsprechend 9 mmol Isobuten) und 0,45 g (3 mmol) Chloressigsäure-tert.-butylester enthielt und im nächsten Ansatz wiederverwendet wurde. Im Sumpf des Rotationsverdampfers blieben 95,4 g hochreine (99,8 Gew%), schwach rot gefärbte Chloressigsäure zurück, die für den nächsten Ansatz wiederverwendet wurden.

In der nachfolgenden Tabelle wird das Ergebnis mit W.S. Johnson et al., J.A.C.S. 75, S. 4998, rechte Spalte (1953) verglichen:

| | Literatur *) | Erfindung |
|---|---|---|
| Raumzeitausbeute [g/l . h] | 27 | 100 |
| Monochloressigsäureumsatz [%] | 59 | 50 |
| Ausbeute, ber. auf umgesetztes Isobuten [%] = Selektivität | 83 | 94 |

| | | |
|---|---|---|
| *) Korrigiert mit dem dort vermerkten Gehalt von 7 % Polyisobuten. | | |

## Patentansprüche

1. Verfahren zur Herstellung von Chloressigsäure-tert.-butylester durch Anlagerung von Chloressigsäure an Isobuten in einem geschlossenen Gefäß unter dem sich einstellenden Druck, dadurch gekennzeichnet, daß man die Reaktion in Abwesenheit von Katalysatoren und Lösemitteln in einem Druckbehälter bei einer Temperatur von 80 ° bis 110 °C unter dem sich einstellenden Druck von 3 bis 12 bar über einen Zeitraum von 1 bis 12, vorzugsweise 4 bis 8 Stunden durchführt, den Druckbehälter abkühlt, das gekühlte Produktgemisch entnimmt und es ohne vorherige Neutralisation unter vermindertem Druck destillativ aufarbeitet.

## Claims

1. A process for preparing tert-butyl chloroacetate by addition of chloroacetic acid to isobutene in a closed vessel under the pressure establishing itself, which comprises carrying out the reaction in the absence of catalysts and solvents in a pressure vessel at a temperature of 80° to 110°C under the pressure establishing itself of 3 to 12 bar over a period of 1 to 12, preferably 4 to 8 hours, cooling the pressure vessel, withdrawing the cooled product mixture and working it up by distillation under reduced pressure without prior neutralization.

## Revendications

1. Procédé de préparation de l'ester tert-butylique de l'acide chloracétique par addition d'acide chloracétique sur de l'isobutène dans un récipient fermé sous la pression qui s'établit, caractérisé en ce que l'on effectue la réaction en l'absence de catalyseurs et de solvants dans un vase sous pression à une température de 80° à 110°C sous la pression de 3 à 12 bars qui s'établit, sur une période de 1 à 12, de préférence de 4 à 8 heures, on refroidit le vase sous pression, on reprend le mélange de produits refroidi et on le traite par distillation sous pression réduite sans neutralisation préalable.
